# FASCICULE DE BREVET EUROPEEN

(11) **EP 2 493 335 B1**
(45) Date de publication et mention de la délivrance du brevet: **24.07.2013**
(21) Numéro de dépôt: 10787824.1
(22) Date de dépôt: 22.10.2010
(51) Int. Cl.: A23L 1/214, A23L 2/02, A23L 2/38, A61K 8/97, A61K 36/9066

(54) **PROCEDE D'OBTENTION D'UN JUS DE RHIZOME DU CURCUMA**
VERFAHREN ZUR GEWINNUNG EINES SAFTES AUS DEM KURKUMA-RHIZOM
METHOD FOR OBTAINING A CURCUMA RHIZOME JUICE

(30) Priorité: 27.10.2009 FR 0957529
(43) Date de publication de la demande: 05.09.2012
(73) Titulaire: Wembi, Lomena, 54600 Villers-les-Nancy (FR); Sonntag, Jean-Claude, 54000 Nancy (FR)
(72) Inventeur: Wembi, Lomena, 54600 Villers-les-Nancy (FR); Sonntag, Jean-Claude, 54000 Nancy (FR)
(74) Mandataire: Poupon, Michel
(86) Numéro de dépôt international: PCT/FR2010/052255
(87) Numéro de publication internationale: WO 2011/051600

(56) Documents cités:
- EP-A2- 0 923 937
- JP-A- 2000 342 242
- JP-A- 2003 111 581
- JP-A- 2009 045 003

## Description

L'invention concerne un procédé d'obtention d'un jus de rhizome du curcuma composé des phytonutriments liposolubles et hydrosolubles contenus dans le rhizome du curcuma frais.

Le rhizome du curcuma frais renferme environ 2 à 6% d'huile essentielle, 45 à 55% d'amidon, une grande quantité d'eau et des curcuminoïdes jusqu'à 8%. L'huile essentielle et les curcuminoïdes sont de plus en plus étudiés et trouvent de multiples applications thérapeutiques.

Les curcumunïodes et l'huile essentielle du curcuma sont des substances aux propriétés anti oxydantes, anti bactériennes, anti fongiques, anti-carcinogènes, anti inflammatoires et anticancéreuses, qui présentent de fait un grand intérêt.

Les composés actifs du curcuma sont généralement extraits de la poudre de curcuma, laquelle poudre est fabriquée à partir des curcumas frais récoltés auxquels on fait subir un long traitement thermique après lavage, épluchage et broyage.

Un procédé d'obtention de jus de curcuma à partir de rhizome de curcuma frais est divulgué dans JP2009 045003A.

Un premier problème lié au procédé connu, traditionnellement utilisé, est une grande détérioration de certains composés actifs contenus dans le rhizome.

Un deuxième problème en relation avec le procédé classique de traitement des rhizomes est le délai important pour l'obtention des matières actives.

Compte tenu par ailleurs des multiples applications des principes actifs contenu dans le curcuma un troisième problème concerne le rendement du procédé de traitement qui est loin d'être optimal, notamment en raison de l'étape d'épluchage des rhizomes.

Le curcuma contient des curcuminoïdes et de l'huile essentielle, compte tenu de l'importance de l'utilisation de ces éléments dans le domaine médical, on a été amené à tenter d'extraire le jus intégral du curcuma, notamment dans le but de réaliser des aliments et boissons diététiques contenant des antioxydants. Toutefois le procédé communément utilisé pour l'obtention du curcuma en poudre constitue un inconvénient majeur pour la conservation de ces micronutriments actifs et pour une optimisation du potentiel thérapeutique du curcuma.

Le procédé connu de traitement du rhizome de curcuma met en oeuvre une étape d'épluchage, compte tenu de la petite taille et de la forme irrégulière du rhizome cette étape induit une perte considérable de matière préjudiciable au rendement global du traitement. Par ailleurs l'épluchage favorise l'oxydation superficielle de la matière devenue accessible à l'oxygène de l'air, ce qui est également en défaveur du procédé.

L'obtention d'un jus de curcuma qui comporte toutes les composantes actives du rhizome d'origine, lesquelles composantes n'étant pas, ou très peu, dénaturées, présente donc des difficultés majeures.

Ce jus de curcuma pourrait constituer un produit nouveau s'il devenait réalisable dans des conditions sanitaires optimales et s'il disposait de tous les composés actifs contenus dans le rhizome de départ.

L'invention a donc pour objectif de résoudre les principales difficultés évoquées précédemment en proposant un procédé de fabrication de jus de curcuma à partir du rhizome du curcuma frais. Le procédé étant caractérisé par le fait qu'il comporte après une étape de lavage du rhizome au moins une étape de congélation puis une étape de décongélation, ledit rhizome n'étant pas épluché, lesquelles étapes de congélation et de décongélation visent à déstructurer le réseau du rhizome de manière à favoriser l'extraction ultérieure des composantes utiles.

Les avantages du procédé de fabrication du jus de curcuma selon l'invention sont multiples :
- il est obtenu directement un jus concentré de curcuma en fin de traitement, sans passage par une phase sèche de la matière,
- les curcuminoïdes et l'huile essentielle ne sont pas dénaturés par le traitement d'extraction, tous les composés actifs du rhizome de curcuma sont récupérés dans le jus,
- le temps nécessaire à l'extraction est nettement amélioré par rapport au procédé connu,
- l'énergie nécessaire pour réaliser le traitement est diminuée,
- le rendement matière est très supérieur à ce qui est obtenu par le traitement traditionnel connu,
- un nouveau produit est fabriqué qui présente de multiples possibilités d'utilisation dans le domaine alimentaire, en cosmétique ou pour des applications pharmaceutiques, thérapeutiques et nutraceutiques.

D'autres caractéristiques et avantages de l'invention se dégageront de la description qui va suivre en regard du dessin annexé qui n'est donné qu'à titre d'exemple non limitatif.

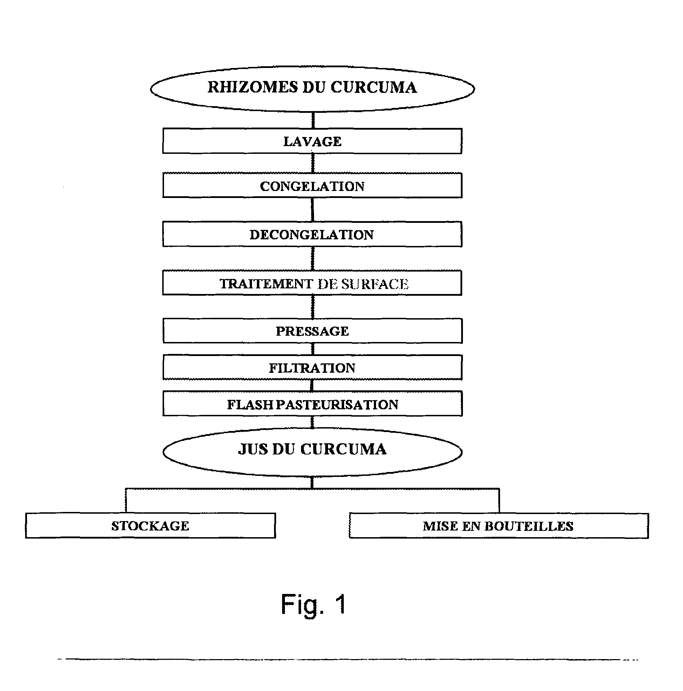

La figure 1 illustre les étapes principales du procédé de fabrication du jus de curcuma selon l'invention.

Comme le montre cette figure le procédé de traitement du rhizome frais de curcuma visant à obtenir un jus de curcuma comporte plusieurs étapes essentielles.

Selon l'invention le procédé comporte au moins une étape de congélation du rhizome suivi d'une étape de décongélation, après le lavage du rhizome.

Il est important de noter que l'étape de congélation est effectuée après une étape de lavage, classique en elle-même, de manière à éliminer la terre et les impuretés présentes dans tout produit biologique provenant directement des exploitations.

Entre l'étape de lavage et la congélation il n'est pas pratiqué d'étape d'épluchage comme cela est réalisé dans le procédé connu de traitement de ces rhizomes. Supprimer cette étape permet d'éviter l'oxydation des produits actifs que l'on cherche à récupérer et améliore fortement le rendement de traitement.

Les étapes de congélation et de décongélation visent à déstructurer le réseau du rhizome de manière à favoriser l'extraction ultérieure des composantes utiles.

Selon l'invention le traitement comporte après les étapes de congélation et de décongélation une étape de traitement de surface visant à réduire la charge bactérienne à la surface du rhizome.

Il peut être envisagé différentes sortes de traitement pour réduire la charge bactérienne à la surface du rhizome, selon une première variante l'étape de traitement de surface est un flash thermique par vapeur ou un échaudage.

Selon une deuxième variante le traitement de surface correspond à une détente instantanée contrôlée.

La détente instantanée contrôlée (DIC) est fondée sur une chute abrupte de pression vers le vide à la suite d'un traitement thermique de courte durée, la DIC comporte donc les étapes suivantes :
- Elévation de température et de la pression : Il s'agit de porter le rhizome à une température comprise entre 100°C et 120°C tout en élevant la pression généralement jusqu'à 4 ou 5 bars pendant quelques secondes seulement.
- Chute abrupte vers le vide : après une phase de stabilisation, le vide est fait dans la chambre du réacteur DIC. C'est sous l'effet de cette dépression vers le vide que la structure moléculaire de la substance est modifiée, le résultat visible est une expansion de la substance traitée, par effet de souffle.
- Retour à la pression et à la température ambiante.

L'ensemble du processus dure moins d'une minute dans la plupart des cas.

La détente instantanée contrôlée offre des avantages économiques, tels que la rapidité de préparation et la réduction du coût de traitement, et des avantages qualitatifs tels que la stérilisation, le maintien des qualités organoleptiques et du contenu en vitamines.

Selon une troisième variante le traitement de surface correspond à un trempage dans de l'eau activée au moyen d'enzymes.

Le trempage du rhizome de curcuma après décongélation dans un bioréacteur pouvant contenir des enzymes immobilisées, et éventuellement dans une préparation enrichie en hydrogène, permet la destruction des bactéries sans augmentation de température. Ce procédé permet le respect de la qualité organoleptique du produit et augment la durée de conservation.

A l'issue de l'étape de traitement de surface, tel que cela est représenté sur la figure 1, il est réalisé une étape de pressage puis une étape de filtration de l'extrait obtenu dans l'étape de pressage.

L'étape de pressage peut être réalisée dans une presse à vis sans fin ou dans tout autre appareil équivalent permettant d'obtenir le jus du curcuma à partir du rhizome déstructuré et traité en surface.

Après obtention du jus filtré il est souhaitable d'effectuer un traitement permettant la conservation du jus de curcuma, le procédé consiste à effectuer une étape avec une flash pasteurisation, une pasteurisation, une stérilisation, ou une combinaison de ces techniques, de manière à détruire complètement tous les micro-organismes encore présents.

L'étape ultime du traitement consistant à stocker ou à mettre en bouteilles dans des conditions adaptées le jus de curcuma prêt à être utilisé.

Avantageusement selon l'invention le jus de curcuma obtenu à l'issue de l'étape de filtration subit une flash pasteurisation avant son conditionnement pour le stockage ou la distribution.

Le jus de curcuma obtenu est enrichi en phytonutriments liposolubles et hydrosolubles, le jus fabriqué peut être mis à profit dans diverses applications, notamment dans le domaine cosmétique, alimentaire, pharmaceutique et thérapeutique.

Dans le domaine alimentaire le jus de curcuma peut être utilisé comme supplément actif à certaines denrées pour réaliser des aliments santé, découlant des propriétés biologiques connues du curcuma.

Dans le domaine cosmétique il est envisagé d'employer le jus de curcuma fabriqué par le procédé de l'invention en association avec des supports traditionnels pour la conception de lotions, de crèmes, de mousses, de savons et divers produits d'hygiène.

Il est décrit également une préparation cosmétique comportant du jus de curcuma obtenu par un procédé de fabrication comportant après une étape de lavage du rhizome frais au moins une étape de congélation puis une étape de décongélation, ledit rhizome n'étant pas épluché, suivi par une étape de traitement de surface.

Dans le domaine pharmaceutique le jus de curcuma permet de récupérer et de concentrer aisément des principes actifs à usages thérapeutiques.

Il est décrit aussi une préparation pharmaceutique et thérapeutique comportant du jus de curcuma obtenu par un procédé de fabrication comportant après une étape de lavage du rhizome frais au moins une étape de congélation puis une étape de décongélation, ledit rhizome n'étant pas épluché, suivi par une étape de traitement de surface.

Par préparation pharmaceutique et thérapeutique il doit être compris toute formulation à usage médical, tout produit ayant pour application la préservation de la santé et tout médicament fabriqué à partir du jus de curcuma, ou d'un composé extrait dudit jus, obtenu par le procédé ici revendiqué.

Le jus de curcuma fabriqué selon le procédé ici décrit peut bien évidemment être modifié par différents additifs introduits à différents stades de la préparation dans le but de modifier le goût, la couleur, la texture ainsi que les fonctionnalités, il peut par exemple être ajouté des agents antioxydants et conservateurs tel que l'acide ascorbique, et divers exhausteurs de goût et colorants.

Le procédé de préparation peut comporter des étapes additionnelles à celles décrites précédemment et différents appareillages peuvent être employés pour réaliser les différentes phases du traitement.

## Revendications

1. Procédé d'obtention de jus de curcuma à partir de rhizome de curcuma frais **caractérisé par le fait qu'**il comporte après une étape de lavage du rhizome au moins une étape de congélation puis une étape de décongélation, ledit rhizome n'étant pas épluché, lesquelles étapes de congélation et de décongélation visent à déstructurer le réseau du rhizome de manière à favoriser l'extraction ultérieure des composantes utiles.

2. Procédé d'obtention de jus de curcuma selon la revendication 1 comportant après les étapes de congélation et de décongélation une étape de traitement de surface visant à réduire la charge bactérienne à la surface du rhizome.

3. Procédé d'obtention de jus de curcuma selon la revendication 2 dans lequel l'étape de traitement de surface est un flash thermique par vapeur ou échaudage.

4. Procédé d'obtention de jus de curcuma selon la revendication 2 dans lequel l'étape de traitement de surface correspond à une détente instantanée contrôlée.

5. Procédé d'obtention de jus de curcuma selon la revendication 2 dans lequel l'étape de traitement de surface correspond à un trempage dans de l'eau activée au moyen d'enzymes.

6. Procédé d'obtention de jus de curcuma selon la revendication 2 dans lequel l'étape de traitement de surface est suivie par une étape de pressage puis par une étape de filtration de l'extrait obtenu dans l'étape de pressage.

7. Procédé d'obtention de jus de curcuma selon la revendication 6 dans lequel le jus de curcuma obtenu à l'issue de l'étape de filtration subit une flash pasteurisation avant son conditionnement pour le stockage ou la distribution.

## Patentansprüche

1. Verfahren zum Gewinnen von Kurkumasaft aus frischem Kurkuma-Rhizom, **dadurch gekennzeichnet, dass** es nach einem Waschschritt des Rhizoms wenigstens einen Gefrierschritt gefolgt von einem Auftauschritt aufweist, wobei das Rhizom nicht geschält ist, wobei die Schritte des Gefrierens und des Auftauens zum Ziel haben, die Struktur des Geflechts des Rhizoms aufzulösen, um das spätere Extrahieren von nützlichen Bestandteilen zu begünstigen.

2. Verfahren zum Gewinnen von Kurkumasaft nach Anspruch 1, das nach den Schritten des Gefrierens und des Auftauens einen Oberflächenbehandlungsschritt aufweist, der zum Ziel hat, die Bakterienbelastung an der Rhizomoberfläche zu verringern.

3. Verfahren zum Gewinnen von Kurkumasaft nach Anspruch 2, bei dem der Oberflächenbehandlungsschritt eine Wärmebehandlung durch Dampf oder Abbrühen ist.

4. Verfahren zum Gewinnen von Kurkumasaft nach Anspruch 2, bei dem der Oberflächenbehandlungsschritt einem kontrollierten schnellen Druckabfall entspricht.

5. Verfahren zum Gewinnen von Kurkumasaft nach Anspruch 2, bei dem der Oberflächenbehandlungsschritt einem Eintauchen in mittels Enzymen aktiviertem Wasser entspricht.

6. Verfahren zum Gewinnen von Kurkumasaft nach Anspruch 2, bei dem auf den Oberflächenbehandlungsschritt ein Pressschritt und anschließend ein Schritt des Filterns des bei dem Pressschritt gewonnenen Extrakts folgt.

7. Verfahren zum Gewinnen von Kurkumasaft nach Anspruch 6, bei dem der am Schluss des Filterschritts gewonnene Kurkumasaft eine Blitz-Pasteurisierung durchläuft, bevor er für die Lagerung oder den Vertrieb verpackt wird.

## Claims

1. Method for obtaining curcuma juice from a rhizome of fresh curcuma, **characterised by** the fact that it comprises at least one freezing step and then a defrosting step after the step of washing the rhizome, said rhizome being unpeeled, the purpose of said freezing and defrosting steps being to break down the structure of the rhizome in order to facilitate the subsequent extraction of useful constituents.

2. Method for obtaining curcuma juice according to claim 1 comprising the step of treating the surface with the aim of reducing the bacterial load on the surface of the rhizome after the freezing and defrosting steps.

3. Method for obtaining curcuma juice according to claim 2, wherein the step of treating the surface is thermal flashing by steaming or scalding.

4. Method for obtaining curcuma juice according to claim 2, wherein the step of treating the surface corresponds to a controlled instantaneous relaxation.

5. Method for obtaining curcuma juice according to claim 2, wherein the step of treating the surface corresponds to soaking in water activated by enzymes.

6. Method for obtaining curcuma juice according to claim 2, wherein the step of treating the surface is followed by a pressing step, then by a step of filtering the extract obtained in the pressing step.

7. Method for obtaining curcuma juice according to claim 6, wherein the curcuma juice obtained after the filtration stage is subjected to flash pasteurisation prior to being packaged for storage or distribution.
